# EUROPEAN PATENT APPLICATION

(11) **EP 1 852 075 A2**
(43) Date of publication of application: **07.11.2007**
(21) Application number: 07251816.0
(22) Date of filing: 30.04.2007
(51) Int. Cl.: A61B 17/34, A61B 17/00, A61B 18/14

(54) **Integrated guidewire needle knife device**

(30) Priority: 01.05.2006 US 381016
(71) Applicant: ETHICON ENDO-SURGERY, INC., Cincinnati, Ohio 45242 (US)
(72) Inventor: Bakos, Gregory J., Mason, OH 45040 (US); Nobis, Rudolph H., Mason OH 45040 (US)
(74) Representative: Tunstall, Christopher Stephen

(57) **Abstract**

Devices for translumenal access are provided. In one embodiment, a device (10) for translumenal access includes an elongate member (12) having proximal and distal ends (12a,12b) and a lumen extending therethrough. A tissue-penetrating wire (14) can extend through the lumen, and in use the wire can be selectively movable between a retracted position where the wire is in a constrained configuration within the lumen and an extended position in which a portion of the wire extends a distance beyond a distal end of the elongate member. The portion of wire which extends the distance beyond the distal end of the elongate member can be adapted to assume a non-linear unconstrained configuration to prevent injury to the tissue and/or tissue structures adjacent to the tissue.

## Description

### FIELD OF THE INVENTION

The present invention relates to surgical devices, and in particular to devices for use in translumenal procedures.

### BACKGROUND OF THE INVENTION

Laparoscopic surgery is one type of minimally invasive surgery in which a surgeon uses numerous trocar ports to access and visualize the tissue site of interest within the abdominal cavity of an anesthetized patient. The benefits of laparoscopic surgery, as compared to open incisional, abdominal surgery, include less pain, shorter recovery time, less scarring, and lower cost. Another way to access the abdominal cavity, however, is via natural openings (mouth, anus, vagina, urethra) of the body and through the peritoneal lining of the abdominal cavity. Obviously, the size and shape of instruments that may be passed through a body lumen in order to perform a medical procedure in the abdominal cavity are greatly restricted due to the anatomical properties of the lumen.

General surgeons, gastroenterologists, and other medical specialists routinely use flexible endoscopes for intralumenal (within the lumen of the alimentary canal) examination and treatment of the upper gastrointestinal (GI) tract, via the mouth, and the lower GI tract, via the anus. In these procedures, the physician pushes the flexible endoscope into the lumen, periodically pausing to articulate the distal end of the endoscope using external control knobs, to redirect the distal tip of the endoscope. In this way, the physician may navigate the crooked passageway of the upper GI past the pharynx, through the esophagus and gastro esophageal junction, and into the stomach. The physician must take great care not to injure the delicate mucosal lining of the lumen, which generally may stretch open to a diameter in the range of about 15-25 mm, but normally has a non-circular cross sectional configuration when relaxed.

During such translumenal procedures, a puncture must be formed in the stomach wall or in the gastrointestinal tract to access the peritoneal cavity. One device often used to form such a puncture is a needle knife which is inserted through the working channel of the endoscope, and which utilizes energy to penetrate through the tissue. A guidewire is then fed through the endoscope and is passed through the puncture in the stomach wall and into the peritoneal cavity. The needle knife is removed, leaving the guidewire as a placeholder. A balloon catheter is then passed over the guidewire and through the working channel of the endoscope to position the balloon within the opening in the stomach wall. The balloon can then be inflated to increase the size of the opening, thereby enabling the endoscope to push against the rear of the balloon and to be fed through the opening and into the peritoneal cavity. Once the endoscope is positioned within the peritoneal cavity, numerous procedures can be performed through the working channel of the endoscope.

While the current methods and devices used to penetrate tissue are effective, one drawback is the risk of damaging adjacent organs and tissue. Due to the low amount of energy and force of penetration needed to pass through tissue, there is the risk of penetrating adjacent tissue that is intended to be left unharmed during the procedure. Accordingly, there remains a need for improved tissue penetrating devices that include a safety feature to protect adjacent tissue. There also remains a need for a simplified procedure that requires fewer steps to form a puncture in tissue.

### SUMMARY OF THE INVENTION

The present invention provides devices and methods for translumenal procedures, and more particularly, for effecting translumenal access. In one aspect, a surgical device is provided that includes an elongate member having proximal and distal ends with a lumen extending therethrough and adapted to be delivered to a surgical site within a patient, and a tissue penetrating wire that is positioned within the lumen. The tissue penetrating wire can be selectively movable between a retracted position in which the wire is in a constrained configuration within the lumen and an extended position in which a portion of the wire extends a distance beyond the distal end of the member. The portion of the wire that extends beyond the distal end of the member can be adapted to assume a non-linear unconstrained configuration. For example, the distal end of the wire can bend, curl, or hang limply when the wire is in the unconstrained configuration. One skilled in the art will appreciate that the wire can be formed from material that is not only conductive, but that is also adapted to assume a predetermined shape when the wire is in the unconstrained configuration. Exemplary materials that can be used to form the wire can include superelastic materials, such as nitinol, shape memory metals, and combinations thereof.

The device can also include a variety of other features to facilitate translumenal access. In one embodiment, the member can include a lock that is adapted to releasably couple the wire to the member to maintain the wire in the extended position. The distal end of the member can also optionally include markings for determining a position of the member relative to the tissue to be penetrated. In other embodiments, the device can be adapted to enlarge the opening formed in the tissue and can include a slit that is formed in a sidewall of the member and a tissue-incising element that is adapted to selectively extend from the slit when it is moved from a constrained configuration to an unconstrained configuration. One exemplary tissue-incising element can include a portion of the wire that is configured to extend a distance away from the longitudinal axis of the member when in the unconstrained configuration.

In another aspect, a method for translumenal access is provided. In one embodiment, the method can include inserting an elongate member to a tissue to be penetrated, selectively advancing a wire out of a lumen of the member to penetrate the tissue, and advancing the member and the wire through the tissue to form an opening in the tissue. Following formation of the opening and upon advancement beyond the lumen, the wire can be adapted to change configuration to prevent injury to structures adjacent to the tissue following formation of the opening. For example, the distal end of the wire can bend, curl, or hang limply upon advancing through the tissue.

The method can further include applying energy to the wire to form the opening in tissue. In one embodiment, the method can include removing the wire from the lumen of the member and delivering an insufflation medium, such as saline, air, or carbon dioxide, from a connector and through the lumen of the member to insufflate a surgical site. In another embodiment, the method can include removing the wire from within the lumen of the member, or alternatively, removing the member from around the wire. A surgical device can then be positioned around an outer circumference of either the member or the wire, such that the device can be inserted along the member or the wire and introduced into the surgical site.

### BRIEF DESCRIPTION OF THE DRAWINGS

The invention will be more fully understood from the following detailed description taken in conjunction with the accompanying drawings, in which:

FIG. 1A is a perspective view of one embodiment of a surgical device for translumenal access;

FIG. 1B is a perspective view of the distal end of the surgical device of FIG. 1A with a wire in an extended position, the wire having a bent distal tip;

FIG. 2 is a perspective view of another embodiment of a distal end of a surgical device with a wire in an extended position, the wire having a curled distal tip;

FIG. 3 is a perspective view of another embodiment of a distal end of a surgical device with a wire in an extended position, the wire having a distal tip that is adapted to become limp;

FIG. 4A is a perspective view of another embodiment of a surgical device for translumenal access, the device having a tissue-incising element;

FIG. 4B is a perspective view of the mid-portion and distal end of the device of FIG. 4A;

FIG. 4C is a perspective view of one embodiment of a tissue-incising element for use with the device of FIG. 4A;

FIG. 5A is a schematic illustrating the device of FIG. 1A prior to insertion into tissue;

FIG. 5B is a schematic illustrating the device of FIG. 1A upon the application of energy to the wire to form an opening in the tissue;

FIG. 5C is a schematic illustrating the device of FIG. 1A following insertion into tissue via the opening formed in FIG. 5B;

FIG. 5D is a schematic illustrating the device of FIG. 1A following removal of the elongate member from the opening;

FIG. 5E is another schematic illustrating the device of FIG. 1A following removal of the elongate member from the opening;

FIG. 6 is a schematic illustrating the device of FIG. 2 following removal of the elongate member from the opening;

FIG. 7 is a schematic illustrating the device of FIG. 3 following removal of the elongate member from the opening;

FIG. 8A is a schematic illustrating one embodiment of a surgical device for translumenal access prior to insertion into tissue, the device including a tissue-incising element;

FIG. 8B is a schematic illustrating the device of FIG. 8A upon the application of energy to the wire to form an opening in the tissue;

FIG. 8C is a schematic illustrating the device of FIG. 8A following insertion into tissue through the opening formed in FIG. 8B and with the tissue-incising element in an exposed position;

FIG. 8D is a schematic illustrating the device of FIG. 8A following the enlargement of the opening formed in FIG. 8B using the tissue-incising element;

FIG. 8E is a schematic illustrating the device of FIG. 8A upon proximal movement of the wire within the lumen such that the wire can exit the elongate member through the slit;

FIG. 8F is another schematic illustrating the device of FIG. 8A upon distal movement of the wire within the lumen such that the wire can exit the elongate member through the slit;

FIG. 8G is a schematic illustrating the device of FIG. 8A where the wire has exited the lumen of the elongate member through the slit; and

FIG. 8H is a schematic illustrating the device of FIG. 8A following removal of the elongate member from the tissue, the wire having a curled distal tip.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention provides devices and methods for translumenal access. In one embodiment, the device can use energy (e.g., electrical current) to create an opening in tissue. Once the opening is created, at least a portion of the device can remain in the tissue to act as a guide device to introduce other surgical devices through the opening to a surgical site. The device can also have features to reduce the likelihood of damaging tissue structures adjacent to the opening formed in the tissue. For example, a wire component of the device can assume a configuration in which it is unlikely to cause inadvertent tissue or organ damage. The devices and methods of the present invention can be used in a variety of surgical procedures that require translumenal access, such as surgeries that require an opening to be formed within a tough-to-penetrate tissue, e.g., the stomach, to facilitate transoral insertion of a treatment device that can be used, for example, in the peritoneal cavity. Exemplary surgical procedures can include transgastric surgeries that require access to the peritoneal cavity through the stomach. The present invention can also be used in conjunction with a variety of devices to form an opening and/or guide a device to a surgical site. By way of non-limiting example, the device can be used in conjunction with a sphinctertome, such as the sphinctertomes disclosed in commonly-owned U.S. Patent Applications entitled "Dual-Bending Sphinctertome" or "Tri-Bending Sphinctertome," both of which are filed concurrently herewith, and the disclosures of which are incorporated by reference herein.

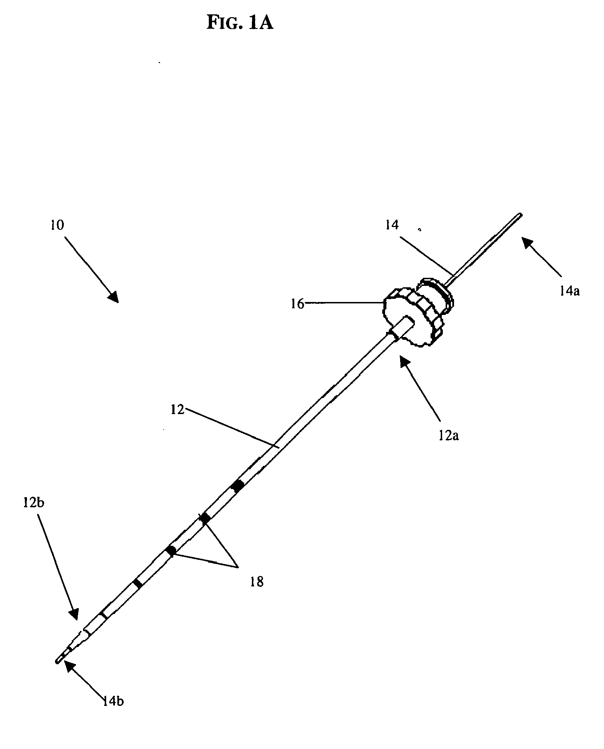

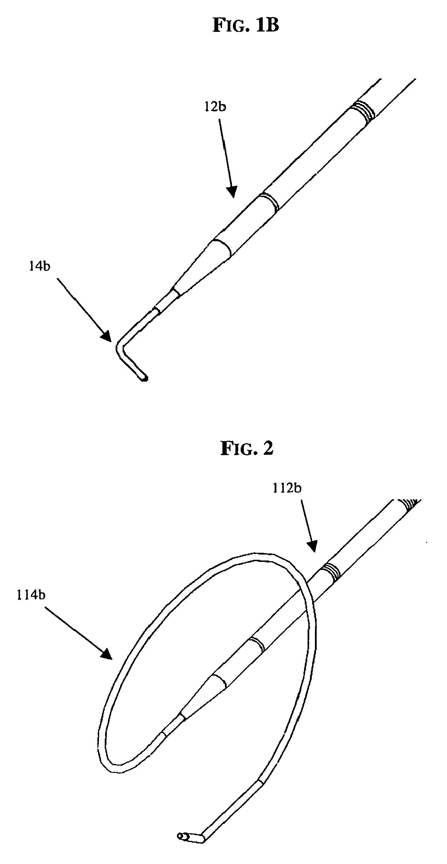

FIGS. 1A-1B illustrate one embodiment of a device 10 for translumenal access. As shown, the device 10 includes an elongate member 12 having proximal and distal ends 12a, 12b, and a lumen (not shown) extending therethrough. A tissue-penetrating wire 14 can extend through the lumen, and the wire 14 can be selectively movable between a retracted position where the wire 14 is held in a constrained configuration within the lumen and an extended position in which a portion of the wire 14 extends a distance beyond a distal end 12b of the elongate member 12. The portion of wire 14 that extends beyond the distal end 12b of the elongate member 12 can be adapted to assume a non-linear configuration in its unconstrained configuration. Such a design is useful to prevent injury to the tissue and/or tissue structures adjacent the opening, as will be discussed in more detail below.

The elongate member 12 can have a variety of shapes and sizes, however it can generally be adapted for insertion and delivery to a tissue to be penetrated via laparoscopic or endoscopic techniques. By way of non-limiting example, the elongate member 12 can be adapted to be positioned within a laparoscopic or endoscopic port to access the peritoneal cavity, and can have an outside diameter in the range of about 1.0 mm to 3.5 mm, and more preferably about 1.5 mm, and an inside diameter in the range of about 0.5 mm to 1.5 mm, and more preferably about 0.75 mm. The lumen that extends through the member can also have a variety of shapes and sizes, and in one embodiment it can have a shape that is complementary to the shape of the wire. For example, in embodiments where the wire 14 has a non-circular cross section, as will be discussed in more detail below, the lumen can also have a non-circular cross section.

The proximal and distal ends 12a, 12b of the elongate member 12 can include features to facilitate manipulation of the wire 14 as well as the insertion of the device 10 into tissue. In one embodiment, the proximal end 12a of the elongate member 12 can include a locking member 16 that is adapted to releasably couple the wire 14 to the member 12 to maintain the wire 14 in a desired position (e.g., the extended position). While a variety of locking members can be used with the device 10 disclosed herein, in an exemplary embodiment the proximal end 12a of the elongate member 12 can include a luer lock 16 that is adapted to couple to a corresponding locking member on the proximal end 14a of the wire 14, as will be discussed in more detail below. Various features can also be included on the proximal end 12a of the elongate member 12 to facilitate movement of the wire. FIG. 1A illustrates the wire 14 extending proximally from the proximal end 12a of the elongate member 12. In use, the position of the wire 14 within the lumen of the elongate member 12 can be adjusted manually, as will be discussed in more detail below. However, in other embodiments the proximal end of the member can include a handle to facilitate movement of the wire. The handle can have a variety of configurations, and one exemplary handle can be substantially elongate and can include features to facilitate grasping by a user, such as a thumb ring. The handle can also include features and components to facilitate actuation of the wire and/or energy delivery to the wire. For example, the handle can include a mechanism that allows slidable movement of the wire, such as a sliding lever or a rotatable knob, to facilitate movement of the wire within the lumen between a retracted position and an extended position. The handle can also include an energy source disposed therein, such as a battery, or it can be adapted to couple to an external energy source, such as a generator or an outlet, and can include a mechanism to effect energy delivery to the wire, such as a button or dial. The proximal end of the elongate member can also optionally include thereon or be adapted to receive a connector. In use, the connector can be coupled to a fluid delivery source to facilitate insufflation of the surgical site, as will be discussed in more detail below. As noted above, the distal end 12b of the elongate member 12 can be adapted to facilitate insertion into tissue, and as shown in FIGS. 1A-1B, the distal end 12b can be tapered and/or rounded. Alternatively or additionally, the distal end 12b of the member 12 can include markings 18 formed thereon to facilitate positioning of the device 10 within the tissue.

One skilled in the art will appreciate that the elongate member 12 can be formed of any biocompatible material that is flexible enough to allow insertion, yet strong enough to maintain the wire in a constrained configuration within the lumen. In an exemplary embodiment, the elongate member 12 can be formed of polyetheretherketone (PEEK). Other suitable materials include polycarbonate, Ultra High Molecular Weight Polyethylene (UHMWPE), or polytetrafluoroethylene (PTFE).

The tissue penetrating wire 14 having proximal and distal ends 14a, 14b can be movably disposed within the lumen of the elongate member 12, and upon movement from within the lumen to outside of the lumen, at least a portion of the wire can be adapted to change shape to prevent tissue damage. In one embodiment, the distal end of the wire 14b can change direction and become non-rigid to avoid puncturing organs or other tissue structures that are adjacent to the opening.

The shape and size of the wire 14 can generally depend upon the wire's intended use, as well as the ability of the elongate member 12 to maintain the wire in the constrained configuration (which is based on the size of the lumen and the stiffness of the member, for example). For example, and as noted above, the wire 14 can have a non-circular cross section, such as a D-shaped cross section. When positioned within a lumen having a complementary shape, the wire 14 can be prevented from rotating. The complementary shape of the lumen can also aid in directing the bending or curling of the wire 14 in a preferred direction. In an exemplary embodiment, the wire 14 can have a thickness in the range of about 0.25 mm to 1.0 mm, and most preferably about 0.45 mm. As noted above, the proximal end 14a of the wire 14 can include a locking member (not shown) that cooperates with the locking member 16 on the proximal end 12a of the elongate member 12 to maintain the wire 14 in the extended position. For example, where the locking member 16 on the member 12 is a luer, the locking member located on the wire 14 can be a female luer receiver that is removably attached to the proximal end of the wire 14a. The proximal end of the wire can also optionally be coupled to an actuation mechanism located within a handle of the device to facilitate proximal and distal movement of the wire within the lumen and/or an energy delivery device located within a handle of the device or external to the device to receive energy. In use, and upon contact with tissue, the energized wire can form an opening in the tissue.

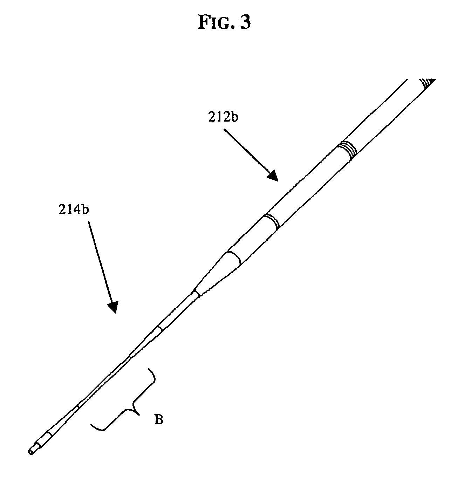

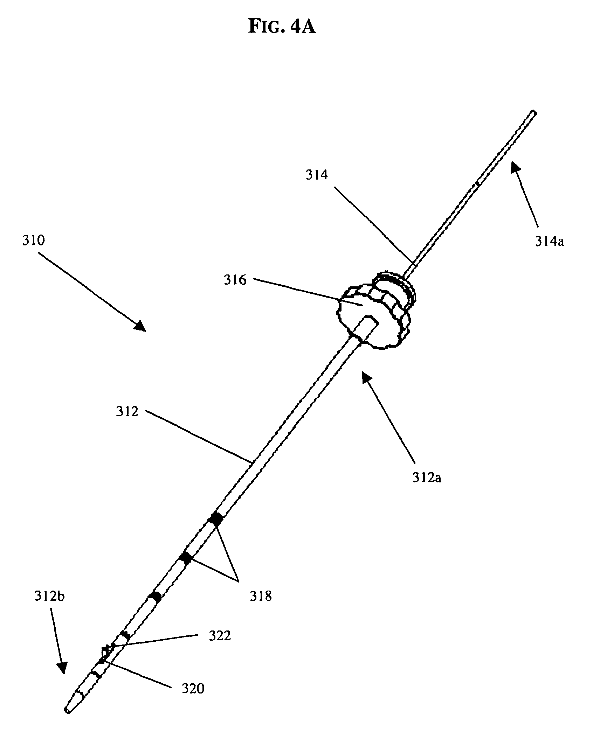

The distal end 14b of the wire 14 can be adapted to change configuration or orientation when the wire is moved from the constrained configuration within the lumen to the unconstrained configuration outside of the lumen. This is effective to prevent damage to the structures adjacent to the tissue. The unconstrained configuration can result in the wire assuming a variety of predetermined configurations or orientations, but the orientation of the wire 14 should be such that it has a trajectory and dimensions that are unlikely to result in unintended puncture of organs or tissue structures adjacent to the opening. For example, and referring back to FIG. 1B, the distal end of the wire 14b can be in the form of a predetermined bend when it is in the unconstrained configuration. That is, a portion of the distal end 14b can be oriented in a direction other than along the longitudinal axis of the portion of the wire 14 constrained within the lumen. As shown, the distal end 14b can be located at an angle relative to the longitudinal axis of the remainder of the wire 14. Alternatively, in FIG. 2, the distal end of the wire 114b can form a predetermined curl or spiral when it is in the unconstrained configuration. That is, the distal end of the wire 114b can be curved relative to the longitudinal axis of the remainder of the wire 114 that is in the constrained configuration within the lumen. In other embodiments, as shown in FIG. 3, at least a portion B of the distal end 214b of the wire 214 can be thinner (and/or made of a more pliable material) than the remainder of the distal portion of the wire 214b. This causes it to flop or hang limply relative to the longitudinal axis of the remainder of the wire 214 when it is in the unconstrained configuration. In each of these embodiments, when the distal end of the wire 14b, 114b, 214b is in the unconstrained configuration, it has characteristics that prevent or minimize the risk of puncturing to the structures adjacent to the opening.

One skilled in the art will appreciate that a variety of materials can be used to form the wire 14, and exemplary materials can be both conductive, to allow the wire to penetrate tissue, and able to change shape when the wire 14 moves from the constrained configuration to the unconstrained configuration. In one embodiment, the wire 14 can be formed of a conductive core material that has an insulating coating surrounding at least a portion of the wire 14. The coating can extend from the proximal end of the wire 14a to a point just proximal to the distal end of the wire 14b, such that the distal end of the wire 14b is uncoated. As a result, the distal end of the wire 14b will conduct energy to the tissue when coupled to an energy source. The wire 14 is long and thin, and by itself has little column strength. However, because of the close fit between the lumen of the member 12 and the outer surface of the wire 14, when the wire 14 is positioned within the lumen of the member 12, penetration through the tissue is possible. When the wire 14 extends a significant distance from an end of the member 12, it will hang limply when the wire 14 is in the unconstrained configuration. This allows the distal end of the wire 14b to effectively change shape when it is moved from the constrained configuration to the unconstrained configuration to a bend, curl, or flop, as noted above. Exemplary unconstrained materials that can be used to form the wire can include superelastic materials, such as nitinol, or a shape memory metals, and exemplary coatings can include Teflon or polyester. The coating can have a wall thickness from about 0.0005 inch to about 0.010 inch, and more preferably about 0.004 inch. While a variety of techniques can be used to form the wire 14, in one embodiment, a heat-shrinkable tubing can be placed over the conductive core material and heated to create the coating.

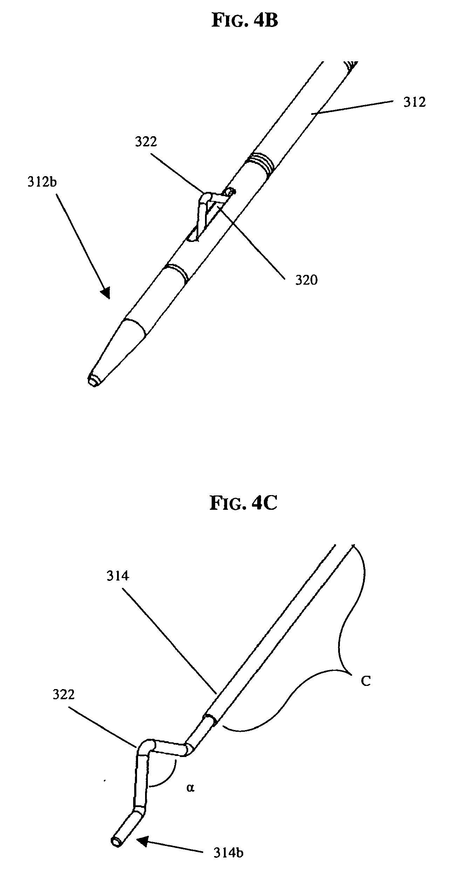

FIGS. 4A-4C illustrate a device 310 for translumenal access that is adapted to enlarge the opening formed within the tissue. The device 310 is similar to the device 10 illustrated in FIGS. 1A-1B, and it includes an elongate member 312 with proximal and distal ends 312a, 312b having a lumen (not shown) extending therebetween. A wire 314 extends through the lumen, and the wire 314 is adapted to be selectively movable from within the lumen to extend a distance beyond the distal end 312b of the member 312. The elongate member 312 can also include a slit 320 located in a sidewall of the member between the proximal and distal ends 312a, 312b thereof from which a tissue-incising element 322 can protrude. Also similar to what is described above, the wire 314 can have a non-circular cross section, for example a D-shape, to facilitate bending or curling in a preferred direction. The lumen in the member 312 can have cross sectional shape that is similar to that of the wire 314, such that the wire 314 cannot rotate within the lumen. The complementary cross sectional shape of the lumen also aids in directing the bending or curling of the wire 314 in a preferred direction, and facilitates alignment of the wire 314 within the lumen of the member 312 to repeatably allow the tissue-incising member 322 to protrude from the slit 320.

The device 310 can also include a variety of other features that can be used to facilitate insertion into tissue. For example, the elongate member 312 can include markers 318 that can be used to help determine the position of the device 310 within the tissue, as well as a locking element 316 located on the proximal end of the member 312a that can mate with a corresponding locking element (not shown) on the wire 314 to hold the wire 314 in the extended position. The device can also optionally include a handle that includes an actuation member, which, by way of example, can have levers or knobs (e.g., three) for moving the wire from the retracted position, to the extended position, and to a third position where the tissue-incising element is extended radially from the member.

In use, and following the formation of the opening in the tissue, the tissue-incising element 322 can be moved from a constrained configuration within the lumen to an unconstrained configuration such that it extends radially outwardly from the slit 320. The device, with the tissue-incising element 322 extended, can then be advanced through the opening such that the opening is expanded to a larger size through the action of the tissue-incising element 322.

The tissue-incising element 322 can have a variety of sizes and configurations effective to enlarge the size of the initial opening. However, in one embodiment, as shown in FIG. 4C, the tissue-incising element 322 can be a portion of the wire 314 that is adapted to extend radially outwardly from the member 312 to effectively increase the cross-sectional size of the member 312. The wire 314 is similar to the wires 14, 114, 214 described above, in that it can include a bent, curled, or floppy distal end portion. Additionally, the wire 314 can include a preconfigured angle α that can function as a tissue-incising element 322 when it is in the unconstrained configuration. While the angle α can have a variety of magnitudes, generally it is in the range of about 60 degrees to about 175 degrees. One skilled in the art will understand that the tissue-incising element 322 can be formed at a variety of locations along the wire 312, and as shown it is located on the distal end 314b of the wire 314.

The wire 314 can also be formed from materials that are similar to the materials noted above, and can have a conductive core material that has an insulating coating surrounding at least a portion of the wire. However, as shown in FIG. 4C, the coating C on the wire 314 can end just proximal to the tissue-incising element 322 formed on the wire 314. This allows the tissue-incising element 322 to conduct energy to the tissue when it moves from the constrained configuration within the lumen to the unconstrained configuration where it is radially extended.

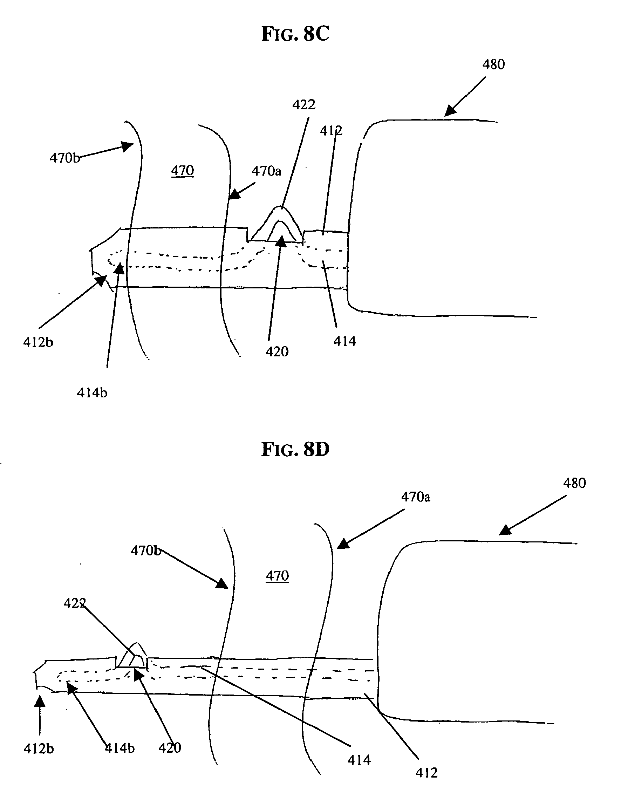

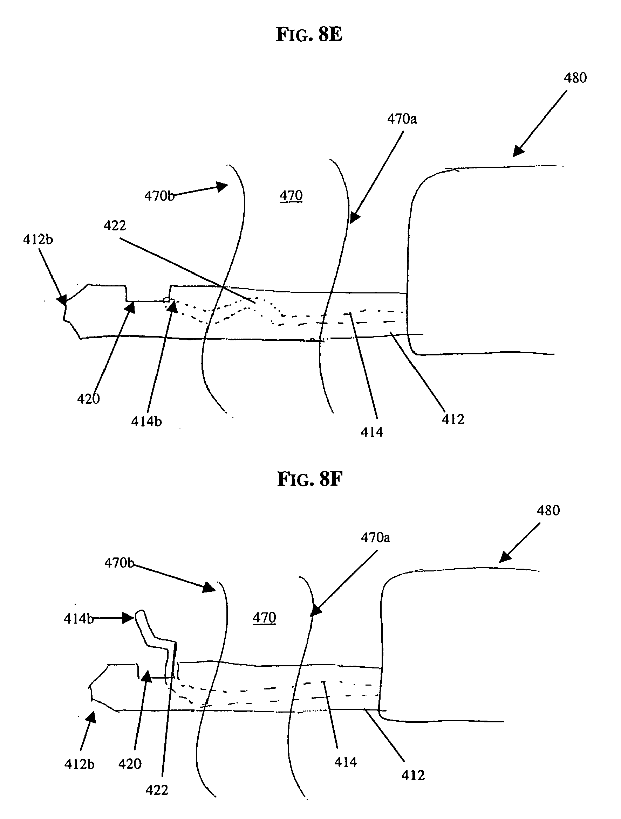

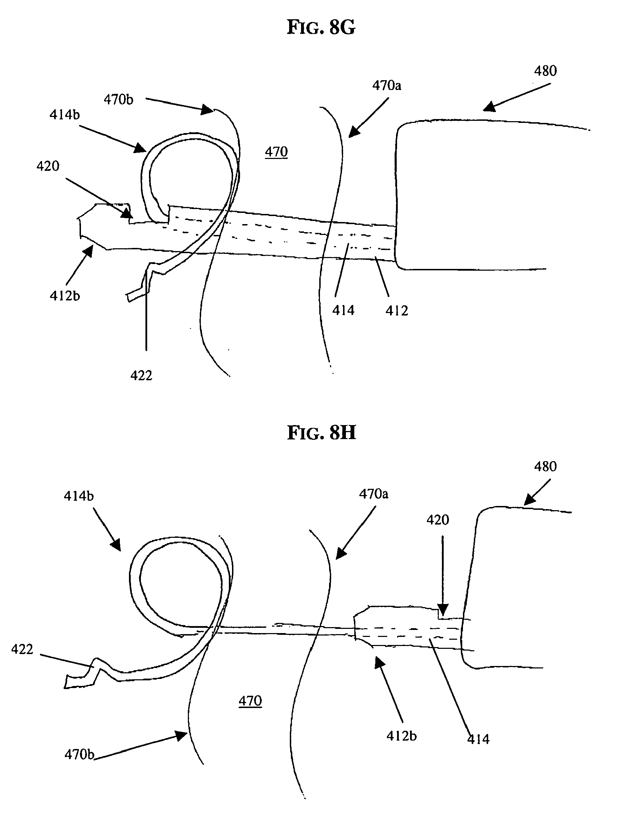

FIGS. 8A-8H illustrate an alternate embodiment of a device 410 for translumenal access that is adapted to enlarge the opening formed within the tissue. The device 410 is similar to the device 310, and includes an elongate member 412 having a lumen 450 extending therethrough. A wire 414 extends through the lumen 450, and the wire 414 is adapted to be selectively movable from within the lumen 450 to extend a distance beyond the distal end 412b of the member 412. The elongate member 412 can also include a slit 420 from which a tissue-incising element 422 can protrude to form an enlarged opening. The tissue-incising element 422 is also similar to the tissue-incising element 322 discussed above, however the tissue-incising element 422 is formed just proximal to the distal end of the wire 414b by a length of about 0.075 inch to about 1.5 inch, more specifically about 0.2 inches). Also similar to the above, the wire 414 can have a non-circular cross section, for example a D-shape, to facilitate bending or curling in a preferred direction. The lumen in the member 414 can have a cross sectional shape that is similar to that of the wire 414 to prevent rotation of the wire 414 within lumen, aid in directing the bending or curling of the wire 414 in a preferred direction, and facilitate alignment of the wire 414 within the lumen of the member 412 to repeatably allow the tissue-incising member 422 to protrude from the slit 420.

One skilled in the art will appreciate that the devices disclosed herein can also be provided as a kit that includes one or more surgical devices, such as diagnostic devices, therapeutic devices, and combinations thereof, that can be inserted over the wire or elongate member to access a surgical site, as will be discussed in more detail below.

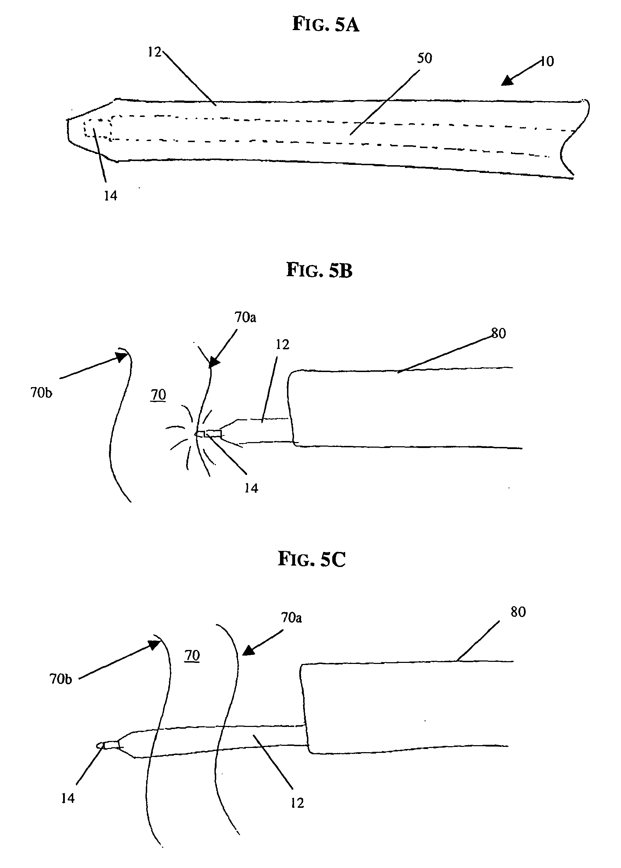

Methods for translumenal access are also provided using the devices disclosed herein. In one embodiment, shown in FIGS. 5A-5E, and following preparation of the patient as known in the art, the elongate member 12 can be inserted via a laparoscopic or endoscopic port 80 and positioned at a tissue 70 to be penetrated. In an exemplary embodiment, the elongate member 12 can be placed through a working channel 80 of a flexible endoscope, and the markers 18 located on the distal end 12b of the member 12 can be used to determine the position of the device 10 within the patient and relative to the tissue 70. When inserted, as shown in FIG. 5A, the wire 12 is in a constrained configuration within the lumen 50 of the member 12 to prevent damage to the port 80. However, once at the tissue 70, the wire 14 can be selectively advanced within the lumen 50 by applying a distal force to the proximal end of the wire 14a while stabilizing the member 12. In alternative embodiments, the wire can be advanced within the lumen by moving an actuation mechanism located on the handle of the device. The wire 14 can be advanced within the lumen 50 to a position such that the locking member on the proximal end of the wire 14a is aligned with the locking member 16 located on the proximal end of the member 12a. The wire 14 can then be locked into position. Alignment and fixation of the corresponding locks indicates that the wire 14 is extended from the distal end 12b of the member 12. This is particularly advantageous in that it reduces the chance that the wire 14 will change position and potentially move back within the lumen 50 as the member 12 and the wire 14 are advanced through the tissue 70.

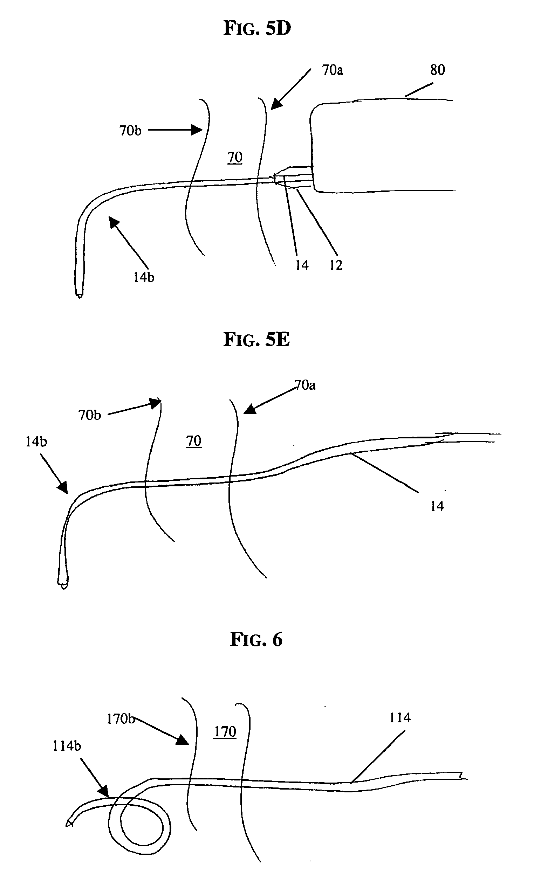

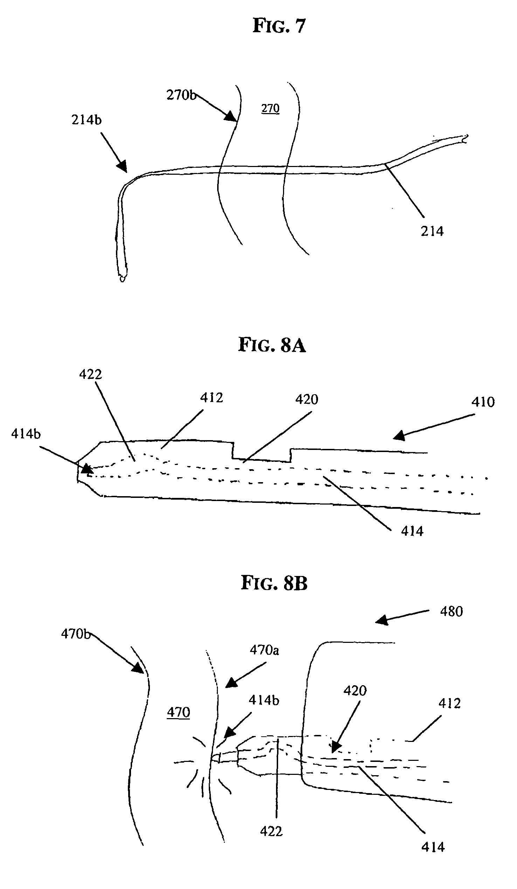

Once the wire 14 is locked in the extended position, energy can be applied to the wire 14 from an energy delivery source. One skilled in the art will appreciate that a variety of forms of energies can be applied to the wire, such as RF energy in either monopolar or bipolar modes. The energized wire 14 and the elongate member 12 can then be advanced from the first side 70a of the tissue 70 to a second side 70b of the tissue 70 to form an opening, as shown in FIG. 5C. As the wire 14 is advanced through the second side 70b of the tissue 70, the distal end 14b of the wire 14 can change configuration to prevent injury to organs or tissue structures adjacent to the opening. For example, as noted above and as shown in FIGS. 5D-7, respectively, the distal end of the wire 14b, 114b, 214b can form a bend, a curl, or hang limply, relative to a longitudinal axis of the remainder of the wire 14, 114, 214 that is constrained within the lumen, once it extends beyond the second side 70b, 170b, 270b of the tissue 70, 170, 270.

After the opening in the tissue 70 is formed, energy delivery to the wire 14 can be terminated. Following removal of the locking members from the wire 14 and the elongate member 12, the device 10 can remain in place to serve as a guide device for other surgical devices, such as diagnostic or therapeutic devices. The other surgical devices can be slid along the outside of the device 10 to pass through the opening to a surgical site. In another embodiment, the wire can be removed from within the lumen of the elongate member, and the elongate member can remain in the tissue to serve as a guide device. Other surgical devices can then be slid along the outside of the member 12 or within the lumen 50 to access a surgical site. Alternatively, and as shown in FIGS. 5E-7 the elongate member can be removed from the tissue 70, 170, 270 while the wire 14, 114, 214 can remain in tissue 70, 170, 270. The wire 14, 114, 214 can then serve as a guide wire, over which a variety of surgical devices can be inserted to access a surgical site.

In other embodiments, the device 10 can be used to facilitate delivery of an insufflation medium to the surgical site to increase the volume of the working space at the surgical site. In one embodiment, the wire 14 can be removed from within the elongate member 12, and a fluid source can then be coupled to the member via a connector. The fluid source can then be activated, and fluid can flow from the source, through the lumen, and into the surgical site. While a variety of fluids can be added to the surgical site, in an exemplary embodiment saline is used. Alternatively, and using a similar technique, air or carbon dioxide can be pumped into the surgical site to effect insufflation. Once the surgical site is insufflated, the guide wire can be reinserted through the lumen of the elongate member and either the wire or the elongate member can be used as a guide. This is particularly advantageous when using a Veress needle with a laparoscopic technique as it creates fluid space within the surgical site. This not only reduces the likelihood of damaging other organs with punctures from the needle, but also allows a surgeon improved visualization during the procedure.

FIGS. 8A-8H illustrate a method for forming an opening of increased size through the tissue 470 using a device 410 having a tissue-incising element 422. Similar to the method illustrated in FIGS. 5A-7, the device 410 can be introduced within a working channel 480 adjacent to the tissue 470. As shown in FIG. 8A, the tissue-incising element 422 and distal end of the wire 414b are located within a constrained configuration within the lumen 450 to prevent damage to the working channel 480 during insertion.

Once the device 410 is inserted into the tissue 470, the distal end 414b of the wire 414 can be advanced from within the lumen 450 to extend a distance beyond the distal end 412b of the member 412. Energy can then be applied to the wire 414, and the wire 414 and the elongate member 412 can penetrate through the first and second sides of the tissue 470a, 470b to form an opening therein, as shown in FIG. 8B. Once the tissue 470 is penetrated, the wire 414 can be moved proximally within the lumen 450. As this happens, the preformed angle of the tissue-incising element 422 protrudes through the slit 420 to effectively increase the cross-sectional dimensions of the member 412, as shown in FIG. 8C. Energy can then be reapplied to the wire 414 and, as shown in FIG. 8D, the device 410 can be advanced through the previously-formed opening in the tissue 470 to cause the opening to increase in size.

Once the opening is formed to a desired size, the wire 414 can then be moved proximally within the lumen. This causes the distal tip 414b to protrude through the slit 420 of the elongate member 412. As the distal tip 414b is moved to an unconstrained configuration, it curls to avoid puncturing adjacent tissue structures. For example, and as noted above, the wire 414 or elongate member 412 can then be removed from the tissue site, and the device 410 can be used as a guide. In alternate embodiments, the wire 414 can be moved proximally within the lumen, such that the tissue-incising element 422 is in the constrained configuration, and the wire 414 and the member 412 can remain in the tissue and can serve as a guide.

Translumenal access devices, including components thereof, can be designed to be disposed after a single use, or they can be designed to be used multiple times. In either case, however, the device or its various components can be reconditioned for reuse after at least one use. Reconditioning can include any combination of the steps of disassembly of the device, followed by cleaning or replacement of particular pieces, and subsequent reassembly. By way of example, the various embodiments of the translumenal access devices described herein can be reconditioned after the device has been used in a medical procedure. The device can be disassembled, and any number of the particular pieces (e.g., the wire, elongate member and/or locking feature) can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a translumenal access device can utilize a variety of techniques for disassembly, cleaning/replacement, and reassembly. Use of such techniques, and the resulting reconditioned translumenal access device, are all within the scope of the present application.

One skilled in the art will further appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A surgical device, comprising:
an elongate member having proximal and distal ends with a lumen extending therethrough and adapted to be delivered to a surgical site within a patient; and
a tissue penetrating wire positioned within the lumen and selectively movable between a retracted position in which the wire is in a constrained configuration within the lumen and an extended position in which a portion of the wire extends a distance beyond the distal end of the member, the portion of the wire extending beyond the distal end assuming a non-linear unconstrained configuration.

2. The device of claim 1, wherein a distal end of the wire includes a bent tip in the unconstrained configuration.

3. The device of claim 1, wherein a distal end of the wire includes a curled tip in the unconstrained configuration.

4. The device of claim 1, wherein a distal end of the wire is adapted to hang limply in the unconstrained configuration.

5. The device of claim 1, wherein at least a distal end of the wire is formed from a material that is adapted to assume a predetermined shape in the unconstrained configuration.

6. The device of claim 5, wherein the material is a superelastic material.

7. The device of claim 6, wherein the superelastic material is nitinol.

8. The device of claim 5, wherein the material is a shape memory metal.

9. The device of claim 1, wherein the wire is made of a conductive material.

10. The device of claim 1, wherein the distal end of the member further comprises markings for determining a position of the member relative to the tissue.
